# EUROPEAN PATENT APPLICATION

(11) **EP 3 511 916 A1**
(43) Date of publication of application: **17.07.2019**
(21) Application number: 17848735.1
(22) Date of filing: 05.09.2017
(51) Int. Cl.: G08B 25/04, G08B 21/02, G08B 25/10

(54) **MONITORING SYSTEM, MONITORING DEVICE, MONITORING METHOD, AND MONITORING PROGRAM**

(30) Priority: 09.09.2016 JP 2016177005
(71) Applicant: Konica Minolta, Inc., Tokyo 100-7015 (JP)
(72) Inventor: NITTA, Kazuma, Tokyo 100-7015 (JP)
(74) Representative: Hoffmann Eitle
(86) International application number: PCT/JP2017/031877
(87) International publication number: WO 2018/047795

(57) **Abstract**

There is provided a watching system capable of suppressing notification of a detection result while notifying a caregiver of a detection result necessary for the caregiver. The watching system (500) includes: an action detection unit (152) capable of detecting a plurality of types of actions of a care recipient; a storage unit for storing notification interval information (124) specifying a notification interval for each combination of actions; a determination unit (154) for determining, when the action detection unit (152) detects that the care recipient takes a first action targeted for notification and, following the first action, detects that the care recipient takes a second action targeted for notification, a notification interval from notification intervals specified in the notification interval information (124), that is associated with a combination of the first action and the second action; and a notification control unit (158) for suppressing notification of the second action when a detection interval from the detection of the first action to the detection of the second action is shorter than the notification interval determined by the determination unit.

## Description

### Technological Field

The present disclosure relates to a technique for watching actions of care recipients who need nursing care such as the elderly and patients.

### Background

A watching system for watching care recipients who need nursing care is increasingly used. The watching system detects that a care recipient gets up, gets out of bed, falls or takes other actions which may be accompanied by danger, and the watching system informs a caregiver accordingly. This allows the caregiver to rush to the care recipient to prevent the care recipient from falling and allow the caregiver to quickly take care of the care recipient.

For a technique for watching a care recipient's actions, Japanese Laid-Open Patent Publication No. 2001-307246 (Patent Literature 1) discloses a human body sensor for "sufficiently coping with various action patterns of a monitored person." Japanese Laid-Open Patent Publication No. 2002-230533 (Patent Literature 2) discloses an image processor capable of accurately determining an event of going to bed and an event of getting out of bed with reference to a boundary side which is a lower side of the geometry of a bed.

### Citation List

### Patent Literature

PTL 1: Japanese Laid-Open Patent Publication No. 2001-307246
PTL 2: Japanese Laid-Open Patent Publication No. 2002-230533

### Summary

### Technical Problem

The human body sensor disclosed in PTL 1 issues notification of any abnormal action of a care recipient that is detected. Similarly, the image processor disclosed in PTL 2 issues notification of any action of a care recipient getting out of bed and going to bed that is detected.

Notifying a caregiver of any action detected increases a burden on the caregiver. Accordingly, there is a demand for a watching system capable of suppressing notification of a detection result while notifying a caregiver of a detection result necessary for the caregiver.

The present disclosure has been made to solve the above problem, and an object thereof in one aspect is to provide a watching system capable of suppressing notification of a detection result while notifying a caregiver of a detection result necessary for the caregiver. An object thereof in another aspect is to provide a watching device capable of suppressing notification of a detection result while notifying a caregiver of a detection result necessary for the caregiver. An object thereof in still another aspect is to provide a watching method capable of suppressing notification of a detection result while notifying a caregiver of a detection result necessary for the caregiver. An object thereof in yet still another aspect is to provide a watching program capable of suppressing notification of a detection result while notifying a caregiver of a detection result necessary for the caregiver.

### Solution to Problem

According to one aspect, a watching system capable of issuing notification of an action of a person to be watched comprises: an action detection unit capable of detecting a plurality of types of actions of the person; a storage unit for storing information specifying a notification interval for each combination of actions; a determination unit for determining, when the action detection unit detects that the person takes a first action targeted for notification and, following the first action, detects that the person takes a second action targeted for notification, a notification interval from notification intervals specified in the information, that is associated with a combination of the first action and the second action; and a notification control unit for suppressing notification of the second action when a detection interval from the detection of the first action to the detection of the second action is shorter than the notification interval determined by the determination unit.

According to another aspect, a watching device capable of issuing notification of an action of a person to be watched, comprises: an action detection unit capable of detecting a plurality of types of actions of the person; a storage unit for storing information specifying a notification interval for each combination of actions; a determination unit for determining, when the action detection unit detects that the person takes a first action targeted for notification and, following the first action, detects that the person takes a second action targeted for notification, a notification interval from notification intervals specified in the information, that is associated with a combination of the first action and the second action; and a notification control unit for suppressing notification of the second action when a detection interval from the detection of the first action to the detection of the second action is shorter than the notification interval determined by the determination unit.

According to another aspect, a watching method capable of detecting a plurality of types of actions of a person to be watched, for issuing notification of a detected action, comprises: preparing information specifying a notification interval for each combination of actions; determining, when the person taking a first action targeted for notification is detected and, following the first action, the person taking a second action targeted for notification is detected, a notification interval from notification intervals specified in the information, that is associated with a combination of the first action and the second action; and suppressing notification of the second action when a detection interval from the detection of the first action to the detection of the second action is shorter than the notification interval determined in the step of determining.

According to another aspect, a watching program is capable of detecting a plurality of types of actions of a person to be watched, and is executed by a computer for issuing notification of a detected action, the watching program causing the computer to perform the steps of: preparing information specifying a notification interval for each combination of actions; determining, when the person taking a first action targeted for notification is detected and, following the first action, the person taking a second action targeted for notification is detected, a notification interval from notification intervals specified in the information, that is associated with a combination of the first action and the second action; and suppressing notification of the second action when a detection interval from the detection of the first action to the detection of the second action is shorter than the notification interval determined in the step of determining.

### Advantageous Effects of Invention

In an aspect, notification of a detection result can be suppressed while notifying a caregiver of a detection result necessary for the caregiver.

These and other objects, features, aspects and advantages of the present disclosure will become apparent from the following detailed description of the invention, taken in conjunction with the accompanying drawings.

### Brief Description of the Drawings

Fig. 1 shows an example of a configuration of an apparatus of a watching system according to a first embodiment.
Fig. 2 shows an input image obtained by capturing an image of a care recipient.
Fig. 3 represents a relationship between a detected action and an action subjected to notification in chronological order.
Fig. 4 shows an example of notification interval information.
Fig. 5 represents a relationship between a detected action and an action subjected to notification in chronological order.
Fig. 6 represents a relationship between a detected action and an action subjected to notification in chronological order.
Fig. 7 shows an example of notification interval information.
Fig. 8 is a block diagram showing an example of a functional configuration of the watching system according to the first embodiment.
Fig. 9 is a conceptual diagram schematically showing a background subtraction process.
Fig. 10 is a flowchart of a process performed by an indoor terminal control device for processing an image.
Fig. 11 is a flowchart of a process performed in Fig. 10 at step S60 for detection of an action.
Fig. 12 is a flowchart of a process performed in Fig. 11 at step S100 for detection of falling.
Fig. 13 is a flowchart of a process performed in Fig. 11 at step S200 for detection of getting up.
Fig. 14 is a flowchart of a process performed in Fig. 11 at step S300 for detection of getting out of bed.
Fig. 15 is a flowchart of a process performed in Fig. 11 at step S400 for detection of stopped breathing.
Fig. 16 shows transition of a screen in the watching system according to the first embodiment.
Fig. 17 shows an example of a main screen.
Fig. 18 shows an example of a setting mode top screen.
Fig. 19 shows an example of an area setting screen.
Fig. 20 shows an example of a normal-time screen.
Fig. 21 shows an example of a notification-time screen.
Fig. 22 is a block diagram showing a main hardware configuration of the watching system according to the first embodiment.
Fig. 23 shows notification interval information referred to by an indoor terminal according to a second embodiment.
Fig. 24 shows notification settings referred to by the indoor terminal according to the third embodiment.

### Detailed Description of Embodiments

Hereafter, reference will be made to the drawings to describe each embodiment of the present invention. In the following description, identical parts and components are identically denoted. Their names and functions are also identical. Accordingly, they will not be described redundantly in detail. Note that each embodiment and each modification described below may be selectively combined as appropriate.

### <First Embodiment>

### [Configuration of Watching System 500]

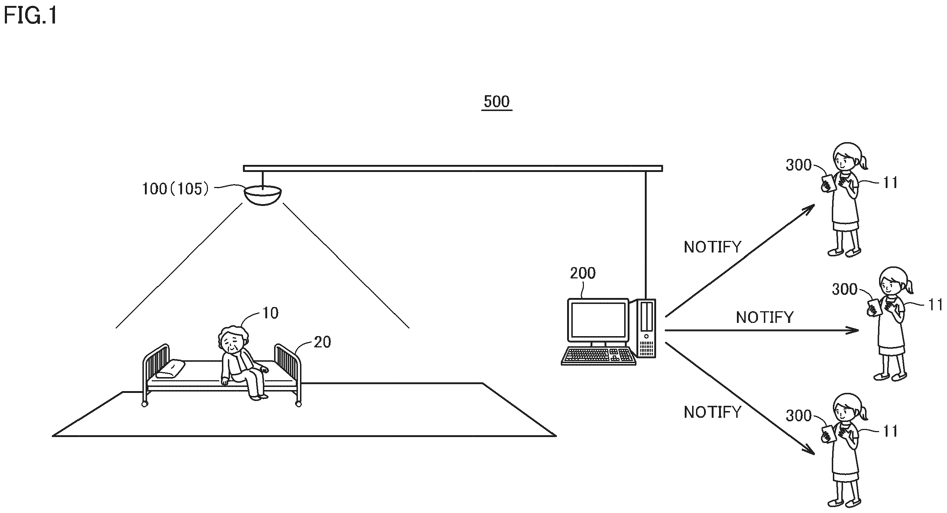

With reference to Fig. 1, a configuration of an apparatus of watching system 500 will be described. Fig. 1 shows an example of a configuration of the apparatus of watching system 500.

Watching system 500 is used, for example, to watch a care recipient 10 who needs nursing care. Care recipient 10 is, for example, an elderly person, a patient, or the like. As shown in Fig. 1, watching system 500 includes an indoor terminal 100 serving as a watching device, a management server 200, and a mobile terminal 300 for a caregiver.

Management server 200 is networked to indoor terminal 100 and mobile terminal 300. Communications between indoor terminal 100 and management server 200 may be implemented through wired communications or wireless communications. When communications between indoor terminal 100 and management server 200 are implemented through wired communications, indoor terminal 100 and management server 200 are connected by, for example, a LAN (Local Area Network) cable. When communications between indoor terminal 100 and management server 200 are implemented through wireless communications, Wifi, Bluetooth (registered trademark), or the like is adopted as standards for wireless communications, for example.

Communications between management server 200 and mobile terminal 300 are implemented through wireless communications. Wifi, Bluetooth, or the like is adopted as standards for wireless communications, for example. Mobile terminal 300 is prepared for a caregiver 11.

Indoor terminal 100 is installed in, for example, a medical facility, a nursing care facility, a house, and the like. Indoor terminal 100 includes a camera 105. Fig. 1 shows care recipient 10 and a bed 20 captured via camera 105 at the ceiling.

Indoor terminal 100 detects various actions of care recipient 10 based on a series of images obtained from camera 105. As an example, actions that can be detected include care recipient 10 getting up, getting out of bed, falling, lying in bed, going to bed, having stopped breathing, etc. An image obtained from camera 105 may be a still image or video.

Upon detecting an action targeted for notification, indoor terminal 100 transmits information indicating the type of the action to management server 200. For example, when getting up is detected as an action targeted for notification, management server 200 notifies mobile terminal 300 of caregiver 11 that care recipient 10 has got up. Mobile terminal 300 is, for example, a smartphone or a tablet terminal. As caregiver 11 is notified via mobile terminal 300 that care recipient 10 has got up, caregiver 11 can help care recipient 10 to rise and get out of bed 20 and can thus prevent care recipient 10 from falling when the care recipient gets out of bed.

While Fig. 1 shows an example in which watching system 500 includes a single indoor terminal 100, watching system 500 may include a plurality of indoor terminals 100. Furthermore, while Fig. 1 shows an example in which watching system 500 includes a single management server 200, watching system 500 may include a plurality of management servers 200. Furthermore, while Fig. 1 shows indoor terminal 100 and management server 200 configured as discrete devices, indoor terminal 100 and management server 200 may be integrally configured.

Furthermore, while Fig. 1 shows an example in which camera 105 is installed on the ceiling, where camera 105 is installed is not limited to the ceiling. It suffices that camera 105 is installed at a place where care recipient 10 can be included in the field of view of camera 105. For example, camera 105 may be installed on a side wall.

### [Action Detection Process by Indoor Terminal 100]

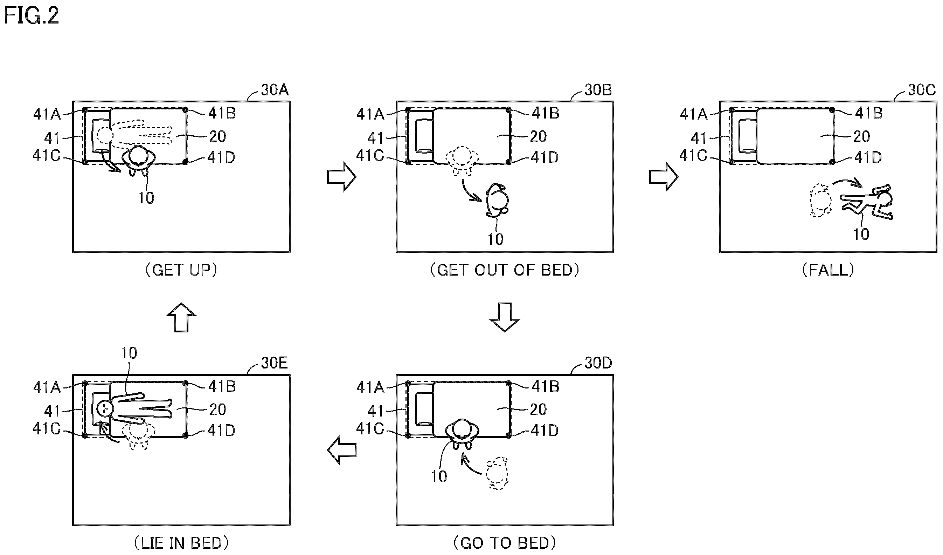

An action detection process by indoor terminal 100 will be described with reference to Fig. 2. Fig. 2 shows input images 30A to 30E obtained by capturing an image of care recipient 10.

Indoor terminal 100 can analyze input images 30A to 30E obtained from camera 105 and detect a plurality of types of actions of care recipient 10. The types of actions that indoor terminal 100 can detect include getting up, getting out of bed, falling, going to bed, and lying in bed, for example. Getting up means that care recipient 10 arises from bed 20. Getting out of bed means that care recipient 10 is away from bed 20. Falling means that care recipient 10 falls on the floor. Going to bed means that care recipient 10 approaches bed 20 before lying down thereon. Lying in bed means that care recipient 10 lies in bed.

As an example, indoor terminal 100 successively detects a person area including care recipient 10 from input images successively obtained from camera 105, and, based on a positional relationship between the location of the person area and a bed area 41, indoor terminal 100 determines an action of care recipient 10. Bed area 41 is preset in a method described later.

More specifically, as shown in input image 30A, based on the fact that the position of the person area has moved from an area inside of bed area 41 to an edge of bed area 41, indoor terminal 100 detects that care recipient 10 has got up. In other words, based on the fact that the person area shifts from a state in which it is included in bed area 41 to a state in which it overlaps an edge of bed area 41, indoor terminal 100 detects that care recipient 10 has got up.

As shown in input image 30B, based on the fact that the position of the person area has moved from the edge of bed area 41 to an area outside of bed area 41, indoor terminal 100 detects that care recipient 10 has got out of bed. In other words, based on the fact that the person area shifts from a state in which it overlaps the edge of bed area 41 to a state in which it does not, indoor terminal 100 detects that care recipient 10 has got out of bed.

As shown in input image 30C, when the position of the person area is outside of bed area 41 and successively detected such that it remains within a prescribed range for a prescribed period of time (for example of 10 seconds) or more, indoor terminal 100 detects that care recipient 10 has fallen. Preferably, when care recipient 10 falls, care recipient 10 moves in an instantaneously increased amount, and accordingly, indoor terminal 100 may add care recipient 10 moving in an instantaneously increased amount as a condition for detecting whether care recipient 10 falls. In other words, when the person area exceeds a prescribed threshold value and thereafter still remains within the prescribed range for the prescribed period of time (for example of 10 seconds) or more, indoor terminal 100 detects that care recipient 10 has fallen.

As shown in input image 30D, based on the fact that the position of the person area has moved from outside of bed area 41 to an edge of bed area 41, indoor terminal 100 detects that care recipient 10 goes to bed. In other words, indoor terminal 100 determines that care recipient 10 goes to bed, based on that the person area has shifted from a state in which it does not overlap the edge of bed area 41 to a state in which it does.

As shown in input image 30E, based on the fact that the position of the person area has moved from the edge of bed area 41 to an area inside of bed area 41, indoor terminal 100 detects that care recipient 10 lies in bed. In other words, based on the fact that the person area shifts from a state in which it overlaps the edge of bed area 41 to a state in which it is included in bed area 41, indoor terminal 100 detects that care recipient 10 lies in bed.

Thus, indoor terminal 100 can detect a plurality of types of actions of care recipient 10 from input images obtained by capturing care recipient 10. The types of actions that indoor terminal 100 can detect are not limited to getting up, getting out of bed, falling, going to bed, and lying in bed. For example, the types of actions that indoor terminal 100 can detect may further include care recipient 10 having stopped breathing. In that case, indoor terminal 100 detects that care recipient 10 has stopped breathing, based on an output of an ultrasonic sensor provided on the ceiling. More specifically, the ultrasonic sensor emits ultrasonic waves to care recipient 10 lying on bed 20, and receives a reflection of the ultrasonic waves. The ultrasonic sensor detects a distance to care recipient 10 based on a period of time elapsing after the emission of the ultrasonic wave before the reception thereof. When care recipient 10 is breathing, the ultrasonic sensor detects a periodically changing distance, whereas when care recipient 10 stops breathing, the ultrasonic sensor detects the distance with periodical change stopped. Noting this point, indoor terminal 100 detects that care recipient 10 has stopped breathing, based on the fact that the distance from the ultrasonic sensor has changed from a periodically changing state to a state remaining within a prescribed range.

In addition, the types of actions that indoor terminal 100 can detect may further include care recipient 10 entering and exiting from a room. In that case, a door area indicating the location of a door is preset in an input image. Based on the person area moving from indoor to the door area, indoor terminal 100 detects that care recipient 10 exits the room, and based on the person area moving from outdoor to the door area, indoor terminal 100 detects that care recipient 10 enters the room.

### [Notification Suppressing Process]

As described above, indoor terminal 100 can detect a plurality of types of actions. An increased number of types of actions detectable by indoor terminal 100 results in a caregiver being notified more frequently and accordingly bearing an increased burden. Accordingly, indoor terminal 100 suppresses notification of a detection result while notifying the caregiver of a detection result necessary for the caregiver.

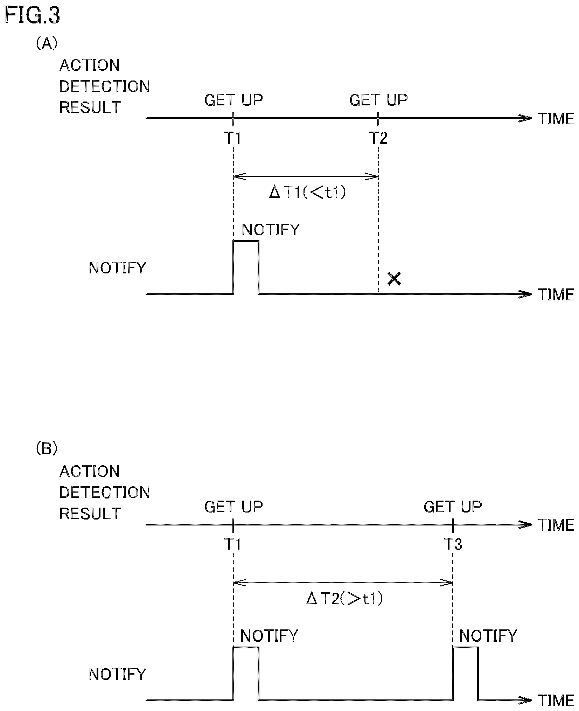

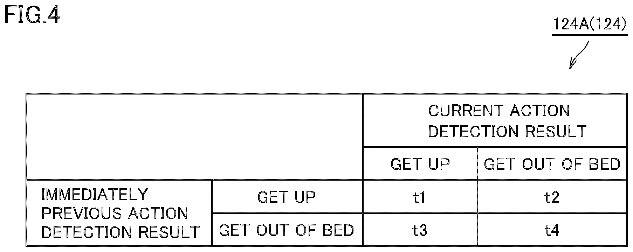

With reference to Fig. 3 and Fig. 4, indoor terminal 100 performs a process for suppressing notification of an action, as described below. Fig. 3 represents a relationship between a detected action and an action subjected to notification in chronological order.

As shown in Fig. 3(A), it is assumed that at time T1, indoor terminal 100 detects "getting up," which is an action targeted for notification. Indoor terminal 100 notifies the caregiver's mobile terminal 300 of the care recipient's action of "getting up."

It is assumed that at time T2, indoor terminal 100 again detects an action of "getting up" targeted for notification. When the caregiver is notified of the action of "getting up" many times, the caregiver would bear an increased burden. Accordingly, based on information which specifies a notification interval for each combination of actions (hereinafter also referred to as "notification interval information"), indoor terminal 100 determines whether notification should be issued for the action of "getting up" detected for the second time.

Fig. 4 shows notification interval information 124A as an example of notification interval information 124. As shown in Fig. 4, notification interval information 124A specifies a notification interval for each combination of a series of actions. The notification interval is a time with reference to which notification is suppressed. That is, when an interval between detected actions is shorter than a specified notification interval, notification of a detected action is suppressed.

Hereinafter, for convenience of illustration, a contextual relationship between detected actions will be represented by an arrow "→." That is, an action preceding the arrow "→" indicates a preceding action, and an action following the arrow "→" indicates a following action. For example, "action A → action B" indicates that action A is performed and an action B is subsequently performed.

As indicated in notification interval information 124A, a combination of "getting up → getting up" is associated with a notification interval t1. A combination of "getting up → getting out of bed" is associated with a notification interval t2. A combination of "getting out of bed → getting up" is associated with a notification interval t3. A combination of "getting out of bed → getting out of bed" is associated with a notification interval t4. Notification intervals t1 to t4 may be preset, or may be changed by the user, as desired.

When indoor terminal 100 detects that a care recipient takes an action of "getting up" (or a first action) targeted for notification and successively detects that the care recipient takes an action of "getting up" (or a second action) targeted for notification, indoor terminal 100 determines from notification intervals t1 to t4 specified in notification interval information 124A notification interval t1 associated with the combination of "getting up → getting up." Notification interval t1 is for example one minute.

As shown in Fig. 3(A), when a detection interval ΔT1 from the detection of the first "getting up" to the detection of the second "getting up" is shorter than notification interval t1, indoor terminal 100 suppresses notification of the second "getting up." Indoor terminal 100 thus notifies the caregiver of the first "getting up" and suppresses notifying the caregiver of the second "getting up," and indoor terminal 100 can thus suppress notification of a detection result while notifying the caregiver of a detection result necessary for the caregiver and thus relieve a burden on the caregiver. In the example of Fig. 3(A), indoor terminal 100 can suppress notification of the same action multiple times for a short period of time.

Suppressing notification includes not only avoiding issuance of notification of an action targeted for notification but also decreasing a level of notification to be lower than that at normal time (that is, when notification of the first "getting up" is issued). Decreasing the level of notification includes, for example, decreasing an audio level of notification to be lower than normal, simplifying the contents of a message for notification, and the like.

As shown in Fig. 3(B), when a detection interval ΔT2 from the detection of the first "getting up" to the detection of the second "getting up" is longer than notification interval t1 determined based on notification interval information 124A, indoor terminal 100 issues notification of the second "getting up." Thus, for a longer detection interval, mobile terminal 300 of the caregiver is notified of an action targeted for notification.

Thus, when indoor terminal 100 detects the first "getting up," indoor terminal 100 issues notification indicating that "getting up" has been detected. Thereafter, when indoor terminal 100 detects the second "getting up," and the detection interval between the first "getting up" and the second "getting up" is longer than notification interval t1, indoor terminal 100 issues notification indicating that the second "getting up" has been detected. In contrast, when indoor terminal 100 detects the second "getting up," and the detection interval between the first "getting up" and the second "getting up" is shorter than notification interval t1, indoor terminal 100 does not issue notification indicating that the second "getting up" has been detected.

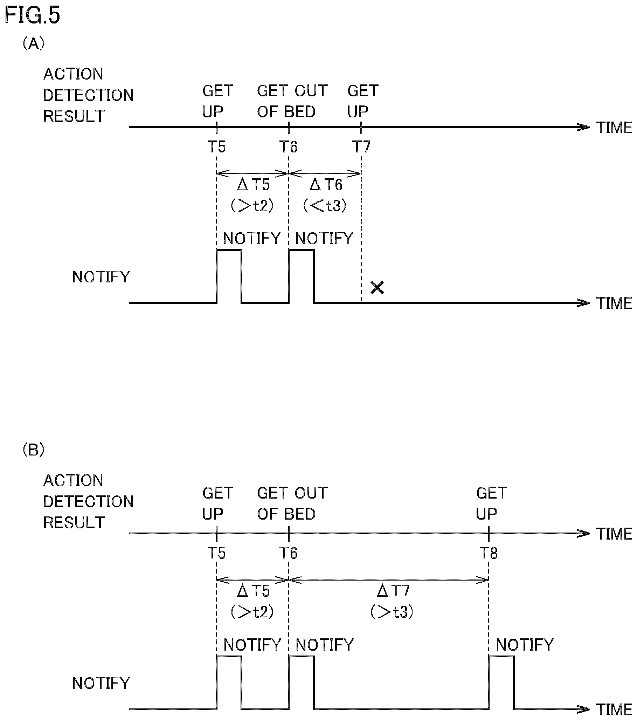

Reference will now be made to Fig. 5 to describe a process for suppressing notification in a case where a detected action is different from that in the example of Fig. 3. Fig. 5 represents a relationship between a detected action and an action subjected to notification in chronological order.

It is assumed that at time T5, indoor terminal 100 detects an action of "getting up" targeted for notification. Since no action has been detected before time T21, indoor terminal 100 notifies mobile terminal 300 of the caregiver that the action of "getting up" has been detected.

It is assumed that at time T6, indoor terminal 100 detects an action of "getting out of bed" targeted for notification. Based on this, indoor terminal 100 determines from notification intervals t1 to t4 specified in notification interval information 124A notification interval t2 associated with "getting up → getting out of bed" corresponding to the combination of the immediately previous action detection result, or "getting up," and the current action detection result, or "getting out of bed." In the example of Figs. 5A and 5B, a detection interval ΔT5 from the detection of the action of "getting up" to the detection of the action of "getting out of bed" is longer than notification interval t2, and indoor terminal 100 notifies the caregiver's mobile terminal 300 of the action of "getting out of bed."

At time T7, it is assumed that indoor terminal 100 detects an action of "getting up." Based on this, indoor terminal 100 determines from notification intervals t1 to t4 specified in notification interval information 124A notification interval t3 associated with "getting out of bed → getting up" corresponding to the combination of the immediately previous action detection result, or "getting out of bed," and the current action detection result, or "getting up."

In the example of Fig. 5(A), a detection interval ΔT6 from the detection of the action of "getting out of bed" to the detection of the action of "getting up" is shorter than notification interval t3, indoor terminal 100 suppresses notification of the action of "getting up." In the example of Fig. 5(B), a detection interval ΔT7 from the detection of the action of "getting out of bed" to the detection of the action of "getting up" is longer than notification interval t3, and indoor terminal 100 notifies the caregiver's mobile terminal 300 of the action of "getting up."

### [Notification Suppressing Process (Modification)]

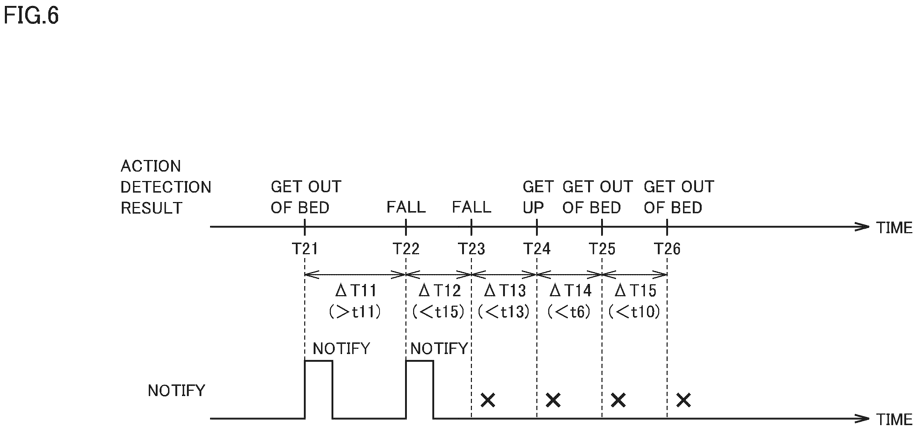

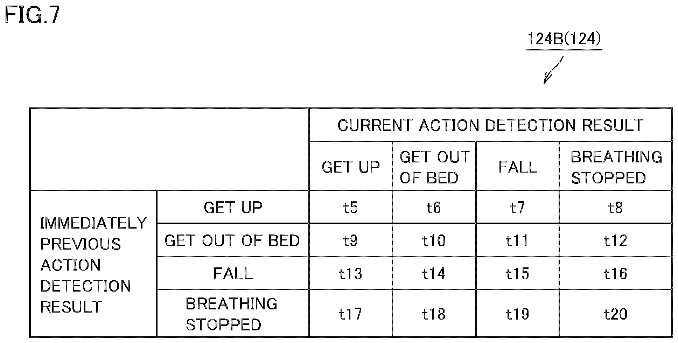

What action is targeted for notification, as specified in notification interval information 124, is not limited to two types of actions. For example, three or more types of actions may be specified in notification interval information 124 as targets for notification. Hereinafter, reference will be made to Fig. 6 and Fig. 7 to describe a notification suppressing process in a case where three or more types of actions are specified in notification interval information 124 as targets for notification.

Fig. 6 represents a relationship between a detected action and an action subjected to notification in chronological order. As shown in Fig. 6(A), it is assumed that at time T21, indoor terminal 100 detects an action of "getting out of bed" targeted for notification. Since no action has been detected before time T21, indoor terminal 100 notifies mobile terminal 300 of the caregiver that the action of "getting out of bed" has been detected.

It is assumed that at time T22, indoor terminal 100 detects an action of "falling" targeted for notification. In response, indoor terminal 100 determines based on notification interval information 124 whether to issue notification for the detected action of "falling."

Fig. 7 shows notification interval information 124B as an example of notification interval information 124. As indicated in notification interval information 124B, a combination of "getting up → getting up" is associated with a notification interval t5. A combination of "getting up → getting out of bed" is associated with a notification interval t6. A combination of "getting up → falling" is associated with a notification interval t7. A combination of "getting up → breathing stopped" is associated with a notification interval t8. A combination of "getting out of bed → getting up" is associated with a notification interval t9. A combination of "getting out of bed → getting out of bed" is associated with a notification interval t10. A combination of "getting out of bed → falling" is associated with a notification interval t11. A combination of "getting out of bed → breathing stopped" is associated with a notification interval t12. A combination of "falling → getting up" is associated with a notification interval t13. A combination of "falling → getting out of bed" is associated with a notification interval t14. A combination of "falling → falling" is associated with a notification interval t15. A combination of "falling → breathing stopped" is associated with a notification interval t16. A combination of "breathing stopped → getting up" is associated with a notification interval t17. A combination of "breathing stopped → getting out of bed" is associated with a notification interval t18. A combination of "breathing stopped → falling" is associated with a notification interval t19. A combination of "breathing stopped → breathing stopped" is associated with a notification interval t20.

A combination of actions which are less likely to occur as a series of actions is associated with a longer notification interval. For example, there is a high possibility that "getting up → getting out of bed" occurs, whereas there is a low possibility that" falling → getting up" occurs. In that case, notification interval t13 associated with "falling → getting up" is set to be longer than notification interval t5 associated with "getting up → getting out of bed." As a result, notification is suppressed for a series of actions which are less likely to occur, and notification is issued for a series of actions which are more likely to occur.

At time T22, based on the fact that an action of "falling" is detected, indoor terminal 100 determines from notification intervals t5 to t20 specified in notification interval information 124B notification interval t11 associated with "getting out of bed → falling" corresponding to the combination of the immediately previous action detection result, or "getting out of bed," and the current action detection result, or "falling." In the example of Fig. 6, a detection interval ΔT11 from the detection of the action of "getting out of bed" to the detection of the action of "falling" is longer than notification interval t11, and indoor terminal 100 notifies the caregiver's mobile terminal 300 of the action of "falling."

It is assumed that at time T23, indoor terminal 100 detects an action of "falling" targeted for notification. Based on this, indoor terminal 100 determines from notification intervals t5 to t20 specified in notification interval information 124B notification interval t15 associated with "falling → falling" corresponding to the combination of the immediately previous action detection result, or "falling," and the current action detection result, or "falling." In the example of Fig. 6, a detection interval ΔT12 from the detection of the immediately previous action of "falling" to the detection of the current action of "falling" is shorter than notification interval t15, and indoor terminal 100 suppresses notification of the currently detected action of "falling."

It is assumed that at time T24, indoor terminal 100 detects an action of "getting up" targeted for notification. Based on this, indoor terminal 100 determines from notification intervals t5 to t20 specified in notification interval information 124B notification interval t13 associated with "falling → getting up" corresponding to the combination of the immediately previous action detection result, or "falling," and the current action detection result, or "getting up." In the example of Fig. 6, a detection interval ΔT13 from the detection of the immediately previous action of "falling" to the detection of the current action of "getting up" is shorter than notification interval t13, and indoor terminal 100 suppresses notification of the currently detected action of "getting up."

It is assumed that at time T25, indoor terminal 100 detects an action of "getting out of bed" targeted for notification. Based on this, indoor terminal 100 determines from notification intervals t5 to t20 specified in notification interval information 124B notification interval t6 associated with "getting up → getting out of bed" corresponding to the combination of the immediately previous action detection result, or "getting up," and the current action detection result, or "getting out of bed." In the example of Fig. 6, a detection interval ΔT14 from the detection of the immediately previous action of "getting up" to the detection of the current action of "getting out of bed" is shorter than notification interval t6, and indoor terminal 100 suppresses notification of the currently detected action of "getting out of bed."

It is assumed that at time T26, indoor terminal 100 detects an action of "getting out of bed" targeted for notification. Based on this, indoor terminal 100 determines from notification intervals t5 to t20 specified in notification interval information 124B notification interval t10 associated with "getting out of bed → getting out of bed" corresponding to the combination of the immediately previous action detection result, or "getting out of bed," and the current action detection result, or "getting out of bed." In the example of Fig. 6, a detection interval ΔT15 from the detection of the immediately previous action of "getting out of bed" to the detection of the current action of "getting out of bed" is shorter than notification interval t10, and indoor terminal 100 suppresses notification of the currently detected action of "getting out of bed."

While notification interval information 124B shown in Fig. 7 specifies a notification interval for each combination of two actions, a notification interval may be specified for each combination of three or more actions. In that case, for a combination of three or more actions, a notification interval may be specified for each interval between the actions or a single notification interval may be specified for the combination of three or more actions.

In one aspect, for a combination of "action A → action B → action C," "action A → action B" is associated with a notification interval tAB and "action B → action C" is associated with a notification interval tBC. When indoor terminal 100 sequentially detects actions A to C, and the detection interval from the detection of action A to the detection of action B is longer than notification interval tAB and the detection interval from the detection of action B to the detection of action C is longer than notification interval tBC, indoor terminal 100 issues notification indicating that action C has been detected. Otherwise, indoor terminal 100 suppresses notification of action C.

In another aspect, the combination of "action A → action B → action C" is associated with a single notification interval t. When indoor terminal 100 sequentially detects actions A to C, and the detection interval from the detection of action A to the detection of action B is longer than notification interval t and the detection interval from the detection of action B to the detection of action C is longer than the same notification interval t, indoor terminal 100 issues notification indicating that action C has been detected. Otherwise, indoor terminal 100 suppresses notification of action C.

### [Functional Configuration of Watching System 500]

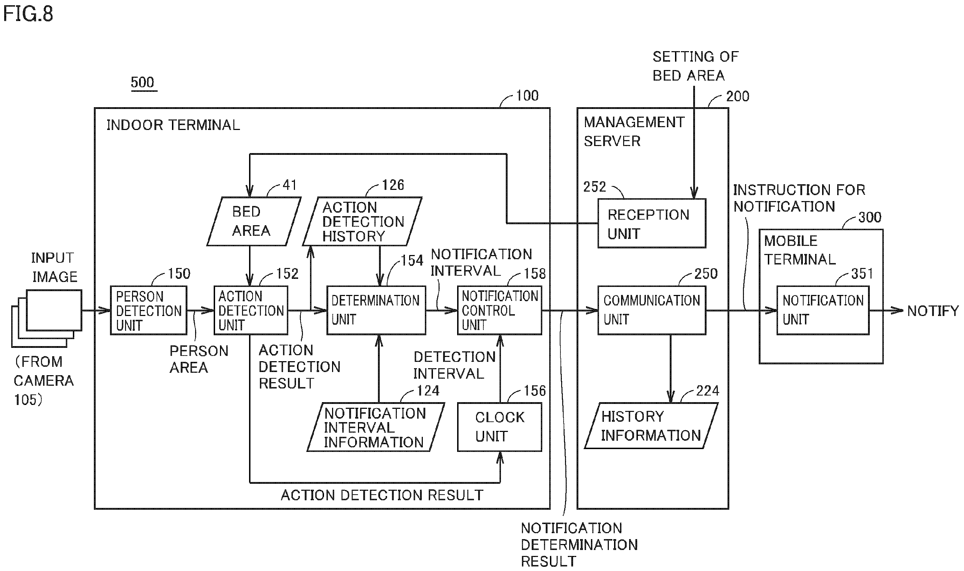

With reference to Fig. 8, a function of watching system 500 will be described. Fig. 8 is a block diagram showing an example of a functional configuration of watching system 500. As shown in Fig. 8, watching system 500 includes indoor terminal 100 serving as a watching device, management server 200, and mobile terminal 300 for a caregiver. In the following, functions of indoor terminal 100, management server 200, and mobile terminal 300 will be sequentially described.

### (Functional Configuration of Indoor Terminal 100)

As shown in Fig. 8, indoor terminal 100 includes a person detection unit 150, an action detection unit 152, a determination unit 154, and a notification control unit 158 as a functional configuration.

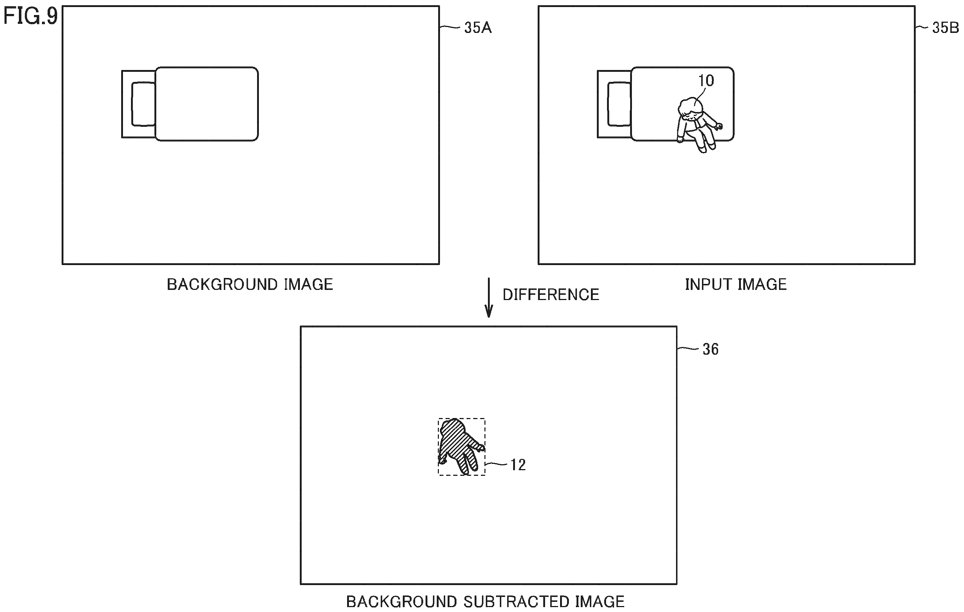

Person detection unit 150 detects a person area including a care recipient from an input image obtained by capturing an image of the care recipient. An example of a person detection process will be described with reference to Fig. 9. For example, person detection unit 150 detects a person area by a background subtraction process. Fig. 9 is a conceptual diagram schematically showing the background subtraction process.

As shown in Fig. 9, person detection unit 150 previously obtains a background image 35A in which there is no image of any person. Person detection unit 150 captures care recipient 10 at the same angle as background image 35A to obtain input image 35B, and subtracts background image 35A from input image 35B. Person detection unit 150 can thus obtain a background subtracted image 36 excluding the background from input image 35B. Person detection unit 150 extracts an area having a pixel value equal to or larger than a prescribed value from background subtracted image 36 and sets a rectangular area circumscribing the extracted area as a person area 12.

Person area 12 is positionally represented by, for example, a coordinate value of a vertex of person area 12 (for example, a coordinate value of an upper left corner thereof) and the area's lateral width and longitudinal length. Alternatively, person area 12 may positionally be represented by coordinate values of two diagonal vertices of person area 12 (for example, upper left and lower right coordinate values thereof).

It should be noted that person area 12 may be extracted in a method different from the method shown in Fig. 9. For example, person detection unit 150 prepares in advance a characteristic portion (that is, a feature value) of care recipient 10 as a template and searches for an area similar to the template by scanning input image 35B. When person detection unit 150 successfully retrieves an area similar to the template within input image 35B, person detection unit 150 sets the area as person area 12. Person detection unit 150 may extract person area 12 using interframe difference, optical flow, tracking, or other image processing techniques.

Referring again to Fig. 8, person area 12 detected is successively output to action detection unit 152. Action detection unit 152 can detect a plurality of types of actions of the care recipient based on the position of person area 12 detected. As an example, action detection unit 152 can detect the care recipient getting up, getting out of bed, going to bed, lying in bed, falling, and the like. These actions are detected in a method which has been described above with reference to Fig. 2, and accordingly, will not be described repeatedly. An action detected by action detection unit 152 is associated with its detection time and thus written in an action detection history 126.

When action detection unit 152 detects that the care recipient takes a first action targeted for notification and, following the first action, detects that the care recipient takes a second action targeted for notification, determination unit 154 determines from the notification intervals specified in notification interval information 124 (see Fig. 4) a notification interval associated with the combination of the first action and the second action. The determined notification interval is output to notification control unit 158.

A clock unit 156 measures a detection interval of actions targeted for notification that are detected by action detection unit 152. More specifically, clock unit 156 measures the detection interval from the detection of the immediately previous action targeted for notification (or the first action) to the current action targeted for notification (or the second action). As an example, clock unit 156 calculates as the detection interval the difference between a time point at which the immediately previous action was detected and that at which the current action is detected. The calculated detection interval is output to notification control unit 158.

When the detection interval measured by clock unit 156 is longer than the notification interval determined by determination unit 154, notification control unit 158 issues notification of the currently detected action. In doing so, notification control unit 158 transmits to mobile terminal 300 notification indicating that an action targeted for notification has been detected. Preferably, notification control unit 158 transmits information representing the immediately previously detected action (or the first action) and information representing the currently detected action (or the second action) together with the notification to mobile terminal 300. For example, notification control unit 158 transmits a series of images including an image representing the immediately previously detected action and an image representing the currently detected action (i.e., video) together with the notification to mobile terminal 300.

On the other hand, when the detection interval measured by clock unit 156 is shorter than the notification interval determined by determination unit 154, notification control unit 158 suppresses notification of the currently detected action. Various suppression methods are adopted as a method for suppressing notification of an action.

In one aspect, notification control unit 158 changes a manner of notification of a detected action depending on whether a detection interval measured by clock unit 156 is longer than a notification interval determined by determination unit 154. That is, the manner of notification of a detected action varies between normal time and when notification is suppressed. As an example, when notification is normally issued, notification indicating that an action targeted for notification has been detected is transmitted to management server 200 together with a video showing that action. In contrast, when notification is suppressed, the video showing the action targeted for notification is not transmitted and notification indicating that the action targeted for notification has been detected is alone transmitted to management server 200.

In still another aspect, depending on whether the detection interval measured by clock unit 156 is longer than the notification interval determined by determination unit 154, notification control unit 158 changes a destination of notification indicating that an action targeted for notification has been detected. That is, a destination of notification is changed between normal time and when notification is suppressed. As an example, when notification is normally issued, notification indicating that an action targeted for notification has been detected is transmitted to mobile terminal 300. Mobile terminal 300 notifies the caregiver that the action targeted for notification has been detected. In contrast, when notification is suppressed, notification indicating that the action targeted for notification has been detected is transmitted to management server 200. Management server 200 records the received notification. That is, in that case, the caregiver is not notified that the action targeted for notification has been detected.

### (Functional Configuration of Management Server 200)

Continuously referring to Fig. 8, a functional configuration of management server 200 will be described. As shown in Fig. 8, management server 200 includes a communication unit 250 and a reception unit 252 as a functional configuration.

Communication unit 250 receives from indoor terminal 100 a notification determination result received from indoor terminal 100. The notification determination result includes information indicating whether to issue notification of a detected action, a manner of notification of the action, a destination of notification of that action, and the like. The notification determination result is written in history information 224. The information written in history information 224 is, for example, identification information (for example, a terminal ID) for identifying indoor terminal 100, the type of an action detected by indoor terminal 100, and the like. In accordance with the notification determination result, communication unit 250 transmits an instruction to mobile terminal 300 for causing it to issue notification of the detected action (hereinafter also referred to as "instruction for notification").

Reception unit 252 receives setting of a bed area with respect to an input image obtained by capturing an image of a room of the care recipient. How the bed area is set will be described later. The set bed area is transmitted to indoor terminal 100.

### (Functional Configuration of Mobile terminal 300)

Continuously referring to Fig. 8, a functional configuration of mobile terminal 300 will be described. As shown in Fig. 8, mobile terminal 300 includes a notification unit 351 as a functional configuration.

In response to an instruction received from management server 200 for notification, notification unit 351 notifies the caregiver that an action targeted for notification has been detected.

In one aspect, notification unit 351 displays on a display of mobile terminal 300 a message indicating that the action targeted for notification has been detected. The message includes the type of the detected action, the name of the care recipient, the room of the care recipient, and the like.

In another aspect, notification unit 351 causes a speaker of mobile terminal 300 to output a sound representing that an action targeted for notification has been detected. The sound includes the type of the detected action, the name of the care recipient, the room of the care recipient, and the like.

### [Control Structure of Watching System 500]

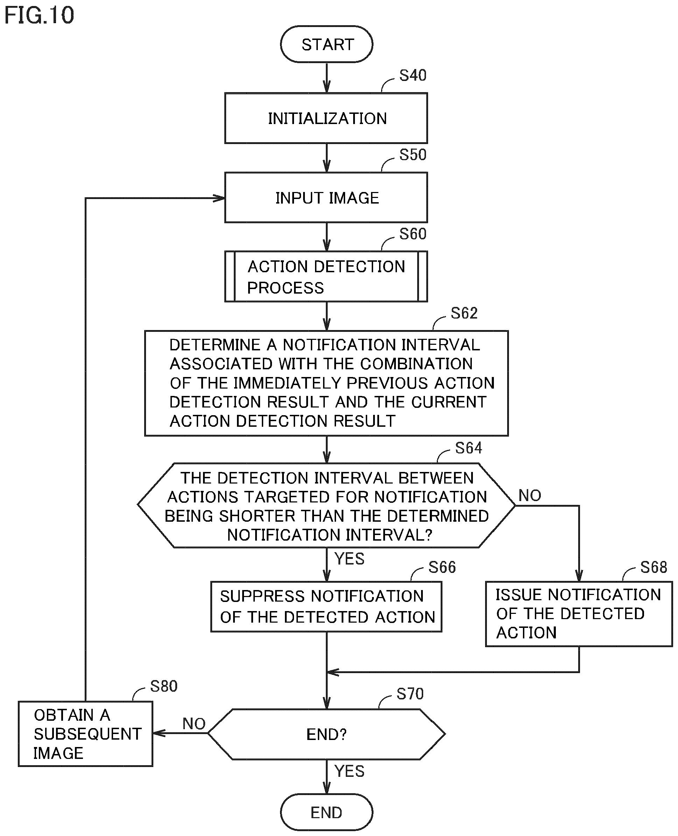

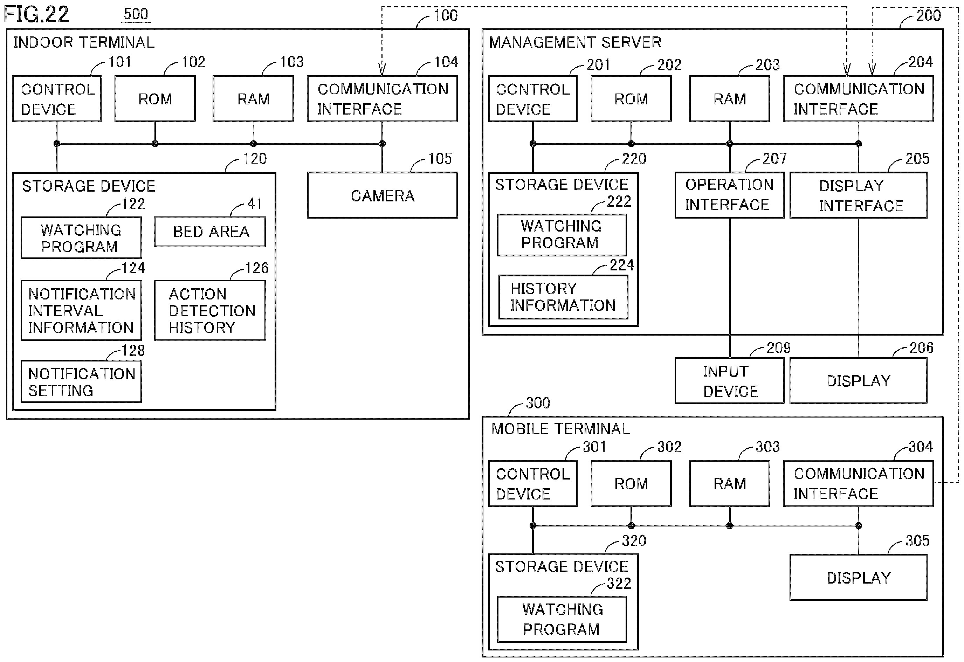

With reference to Fig. 10, a control structure of watching system 500 will be described. Fig. 10 is a flowchart of a process performed by control device 101 of indoor terminal 100 (see Fig. 22) to process an image. The process in Fig. 10 is performed by a computer of indoor terminal 100 or the like, for example. In another aspect, the process may partially or entirely be performed by control device 201 of management server 200 (see Fig. 22), circuit elements, and other hardware.

In step S40, control device 101 performs initialization based on execution of the watching program according to the present embodiment.

In step S50, control device 101 inputs to the watching program according to the present embodiment an input image obtained by capturing an image of a care recipient to be watched.

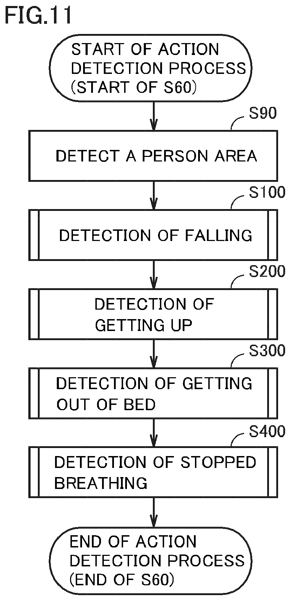

In step S60, control device 101 as action detection unit 152 described above (see Fig. 8) detects that the care recipient takes an action targeted for notification. The flow of the action detection process will be described later (see Fig. 11).

In step S62, control device 101 as determination unit 154 described above (see Fig. 8) determines from the notification intervals specified in notification interval information 124 described above (see Fig. 4) the notification interval associated with the combination of an action targeted for notification immediately previously detected and an action targeted for notification currently detected.

In step S64, control device 101 as notification control unit 158 described above (see Fig. 8) determines whether the detection interval from a time point at which the immediately previously detected action was detected to a time point at which the currently detected action is detected is shorter than the notification interval determined in step S62. When control device 101 determines that the detection interval is shorter than the notification interval determined in step S62 (YES in step S64), control device 101 proceeds to step S66. Otherwise (NO in step S64), control device 101 proceeds to step S68.

In step S66, control device 101 as notification control unit 158 suppresses notification of the detected action. How the notification is suppressed is not particularly limited. For example, the notification may be suppressed by prohibiting the notification or may be suppressed by decreasing the level of notification to be lower than that at normal time.

In step S68, control device 101 as notification control unit 158 notifies the caregiver's mobile terminal 300 of the detected action.

In step S70, control device 101 determines whether to end the watching process according to the present embodiment. For example, control device 101 determines that the watching process according to the present embodiment ends when an administrator performs an operation to terminate the process. When control device 101 determines that the watching process according to the present embodiment ends (YES in step S70), control device 101 ends the process shown in Fig. 10. Otherwise (NO in step S70), control device 101 proceeds to step S80.

In step S80, control device 101 obtains a subsequent input image from camera 105 (see Fig. 1). By repeating step S80, temporally successive input images are successively obtained.

### [Action Detecting Flow]

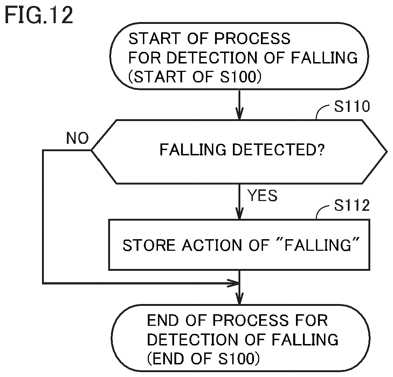

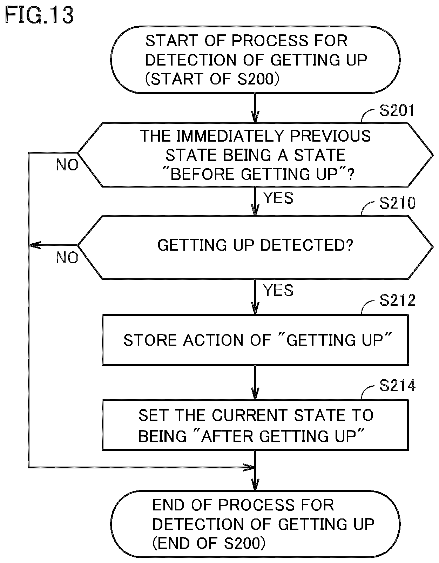

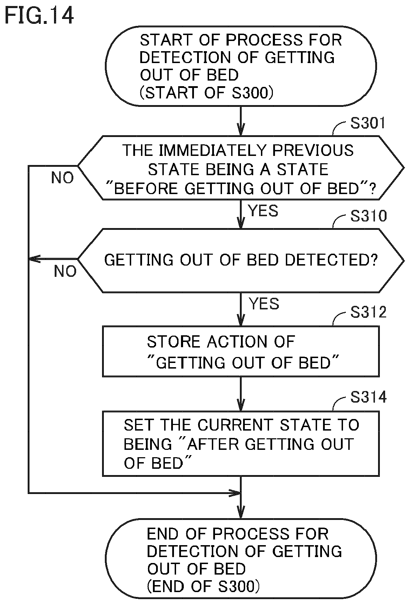

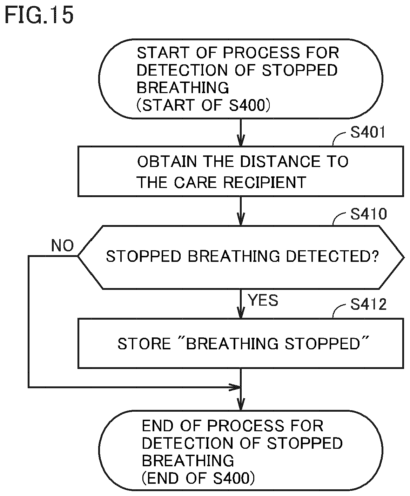

With reference to Figs. 11 to 15, the action detection process performed in step S60 of Fig. 10 will be described in detail. Fig. 11 is a flowchart of a process performed in Fig. 10 at step S60 for detection of an action. Fig. 12 is a flowchart of a process performed in Fig. 11 at step S100 for detection of falling. Fig. 13 is a flowchart of a process performed in Fig. 11 at step S200 for detection of getting up. Fig. 14 is a flowchart of a process performed in Fig. 11 at step S300 for detection of getting out of bed. Fig. 15 is a flowchart of a process performed in Fig. 11 at step S400 for detection of stopped breathing.

Referring to Fig. 11, in step S90, control device 101 as person detection unit 150 described above (see Fig. 8) detects a person area in an input image. How the person area is detected has previously been described with reference to Fig. 9, and accordingly, will not be described.

In step S100, control device 101 performs the process for detecting whether a care recipient has fallen. With reference to Fig. 12, the process for detection of falling will be described.

In step S110, control device 101 determines whether the care recipient falling is detected. As one example, when the position of the person area in the input image is outside the bed area and successively detected such that it remains within a prescribed range for a prescribed period of time (for example of 10 seconds) or more, indoor terminal 100 determines that care recipient 10 falling is detected. When control device 101 determines that the care recipient falling is detected (YES in step S110), control device 101 proceeds to step S112. Otherwise (NO in step S110), control device 101 ends the fall detection process shown in Fig. 12.

In step S112, control device 101 associates the care recipient's action of "falling" with the time at which the action was detected, and thus writes the action with the time in action detection history 126 (see Fig. 8). Preferably, control device 101 further writes a video showing the care recipient's action of "falling" in action detection history 126.

Referring again to Fig. 11, in step S200, control device 101 performs a process for detecting the care recipient getting up. With reference to Fig. 13, the process for detection of getting up will be described.

In step S201, control device 101 determines whether the immediately previous action detection result is an action "before getting up." As an example, the action "before getting up" is the care recipient "lying in bed." When control device 101 determines that the care recipient is in a state "before getting up" (YES in step S201), control device 101 proceeds to step S210. Otherwise (NO in step S201), control device 101 ends the process for detection of getting up shown in Fig 13.

In step S210, control device 101 determines whether the care recipient getting up is detected. As one example, based on the fact that the position of the person area in an input image has moved from inside of the bed area to an edge of the bed area, control device 101 determines that the care recipient getting up is detected. When control device 101 determines that the care recipient getting up is detected (YES in step S210), control device 101 proceeds to step S212. Otherwise (NO in step S210), control device 101 ends the process for detection of getting up shown in Fig. 13.

In step S212, control device 101 associates the care recipient's action of "getting up" with the time at which the action was detected, and thus writes the action with the time in action detection history 126 (see Fig. 8). Preferably, control device 101 further writes a video showing the care recipient's action of "getting up" in action detection history 126.

In step S214, control device 101 sets the current state of the care recipient to being "after getting up."

Referring again to Fig. 11, in step S300, control device 101 performs a process for detecting the care recipient getting out of bed. With reference to Fig. 14, the process for detection of getting out of bed will be described.

In step S301, control device 101 determines whether the immediately previous action detection result is an action "before getting out of bed." As an example, the action "before getting out of bed" is the care recipient "getting up." When control device 101 determines that the care recipient is in a state "before getting out of bed" (YES in step S301), control device 101 proceeds to step S310. Otherwise (NO in step S301), control device 101 ends the process for detection of getting out of bed shown in Fig 14.

In step S310, control device 101 determines whether the care recipient getting out of bed is detected. As one example, based on the fact that the position of the person area in an input image has moved from an edge of the bed area to outside the bed area, control device 101 determines that the care recipient getting out of bed is detected. When control device 101 determines that the care recipient getting out of bed is detected (YES in step S310), control device 101 proceeds to step S312. Otherwise (NO in step S310), control device 101 ends the process for detection of getting out of bed shown in Fig. 14.

In step S312, control device 101 associates the care recipient's action of "getting out of bed" with the time at which the action was detected, and thus writes the action with the time in action detection history 126 (see Fig. 8). Preferably, control device 101 further writes a video showing the care recipient's action of "getting out of bed" in action detection history 126.

In step S314, control device 101 sets the current state of the care recipient to being "after getting out of bed."

Referring again to Fig. 11, in step S400, control device 101 performs a process for detecting the care recipient having stopped breathing. With reference to Fig. 15, the process for detection of stopped breathing will be described.

In step S401, control device 101 obtains from an ultrasonic sensor installed on the ceiling over the bed the distance from the sensor to the care recipient.

In step S410, control device 101 determines whether the care recipient having stopped breathing is detected. When the care recipient is breathing, the distance obtained in step S401 periodically changes, whereas when the care recipient has stopped breathing, the distance's periodical change stops. Noting this point, control device 101 determines that care recipient 10 having stopped breathing is detected, based on the fact that the distance from the ultrasonic sensor has changed from a periodically changing state to a state remaining within a prescribed range. When control device 101 determines that the care recipient having stopped breathing is detected (YES in step S410), control device 101 proceeds to step S412. Otherwise (NO in step S310), control device 101 ends the process for detection of stopped breathing shown in Fig 15.

In step S412, control device 101 associates the care recipient's action of "having stopped breathing" with the time at which the action was detected, and thus writes the action with the time in action detection history 126 (see Fig. 8). Preferably, control device 101 further writes a video showing the care recipient's action of "having stopped breathing" in action detection history 126.

### [Transition of Screen of Watching System 500]

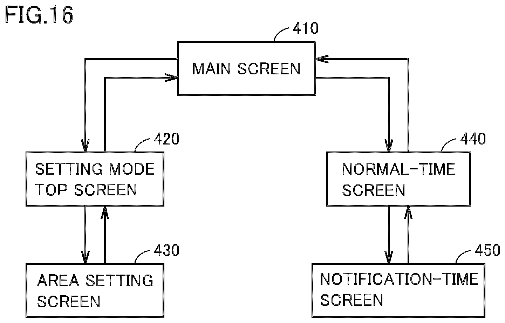

An example of a screen displayed on watching system 500 will be described with reference to Figs. 16 to 21. Fig. 16 is a diagram showing transition of a screen of watching system 500.

When the watching program according to the present embodiment is executed, watching system 500 displays a main screen 410 as an initial screen. From main screen 410, an administrator can make a transition to a setting mode top screen 420 or a normal-time screen 440. From setting mode top screen 420, the administrator can make a transition to main screen 410 or an area setting screen 430. From area setting screen 430, the administrator can make a transition to setting mode top screen 420. From normal-time screen 440, the administrator can make a transition to main screen 410 or a notification-time screen 450. From notification-time screen 450, the administrator can make a transition to normal-time screen 440.

Main screen 410, setting mode top screen 420, area setting screen 430, normal-time screen 440, and notification-time screen 450 are displayed on, for example, a display of management server 200 or a display of mobile terminal 300 for a caregiver.

Hereinafter, examples of main screen 410, setting mode top screen 420, area setting screen 430, normal-time screen 440, and notification-time screen 450 will be described.

### (Main Screen 410)

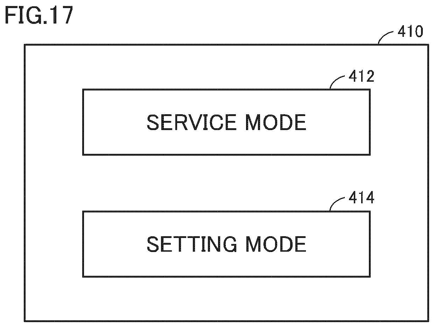

Fig. 17 shows an example of main screen 410. When the watching program according to the present embodiment is executed, watching system 500 displays main screen 410 as an initial screen.

Main screen 410 includes a button 412 for accepting starting the action detection process and a button 414 for opening a setting screen for the action detection process. When watching system 500 detects that button 412 is pressed, watching system 500 displays normal-time screen 440. In addition, when watching system 500 detects that button 414 is pressed, watching system 500 displays setting mode top screen 420.

### (Setting Mode Top Screen 420)

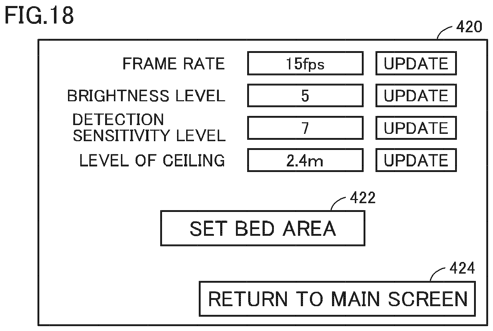

Fig. 18 shows an example of setting mode top screen 420. Setting mode top screen 420 is displayed when watching system 500 is initialized, undergoes maintenance, and the like.

Setting mode top screen 420 receives a setting of a parameter for the action detection process. For example, setting mode top screen 420 receives a parameter for a frame rate for camera 105 (see Fig. 1). Further, setting mode top screen 420 receives a parameter in brightness for an image output from camera 105. Further, setting mode top screen 420 receives a parameter in sensitivity for detecting an action of a care recipient. Furthermore, setting mode top screen 420 receives a parameter for the height of the ceiling on which camera 105 is installed. When an "update" button on setting mode top screen 420 is pressed, each parameter is reflected in watching system 500.

When watching system 500 detects that button 422 is pressed, watching system 500 displays area setting screen 430. When watching system 500 detects that button 424 is pressed, watching system 500 displays main screen 410.

Setting mode top screen 420 may receive other parameters input. For example, setting mode top screen 420 may receive as parameters for camera 105 a parameter for the contrast of the input image, a parameter for adjustment of zooming of the camera, a parameter for adjustment of panning/tilting of the camera, and the like. In addition, setting mode top screen 420 may receive a compression ratio of an image to be transmitted from indoor terminal 100 to watching system 500 and the like. Alternatively, setting mode top screen 420 may receive settings such as time zones for discriminating actions such as getting up, going to bed, and the like.

### (Area Setting Screen 430)

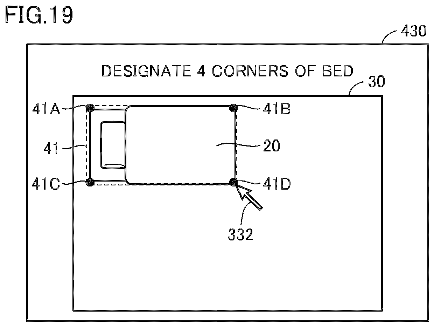

Fig. 19 shows an example of area setting screen 430. Area setting screen 430 receives a setting of bed area 41 for input image 30. As described above, bed area 41 having been set is used in the action detection process.

Area setting screen 430 receives a setting of bed area 41 by receiving a setting of points 41A to 41D, for example. As an example, points 41A to 41D are input via a pointer 432 ganged with an operation of a mouse. In response to an operation received to store bed area 41 set by the administrator, watching system 500 stores information (for example, a coordinate value or the like) for specifying bed area 41 in input image 30.

While in Fig. 19 an example in which points 41A to 41D are set has been described as a method for setting bed area 41, bed area 41 may be set in a different method. For example, area setting screen 430 may receive a setting of bed area 41 by receiving a setting of a line. As another different method, area setting screen 430 receives a setting of bed area 41 by receiving a setting of a plane. In that case, the administrator performs a dragging operation on area setting screen 430 to designate a range in which bed 20 is shown. Thus, as a method for setting bed area 41, any method capable of designating partially or entirely a boundary of the bed area and another area can be adopted.

Further, while in Fig. 19 as a method for setting bed area 41 an example of setting a rectangular boundary has been described, bed area 41 may be set in a different form. For example, bed area 41 may be set in other forms such as a circle, an ellipse, a polygon (for example, a hexagon). Alternatively, bed area 41 may be represented by a line, an arc or the like. The line and the arc may be prescribed in thickness.

Further, while in Fig. 19 an example of setting bed area 41 with pointer 432 has been described, bed area 41 may be set via a different operation such as a touch operation.

Further, while in Fig. 19 an example of setting bed area 41 for bed 20 has been described, an object for which an area is set is not limited to a bed. For example, the object for which the area is set includes bedding such as bedclothes, a door, a chair, and other objects used by the care recipient.

Further, while in Fig. 19 an example in which bed area 41 is manually set by an administrator has been described, watching system 500 may automatically detect bed area 41 by image processing such as edge extraction or template matching. Alternatively, watching system 500 may detect bed area 41 via a 3D sensor, a position sensor attached to a leg of bed 20, a carpet having a pressure sensor, and/or other sensors.

### (Normal-Time Screen 440)

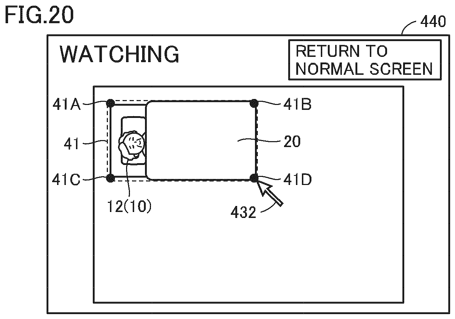

Fig. 20 shows an example of normal-time screen 440. Normal-time screen 440 is a screen displayed when care recipient 10 to be monitored is taking an action which is not dangerous (for example, being asleep in bed) while watching system 500 performs the action detection process. As an example, watching system 500 exactly displays on normal-time screen 440 an image (or video) obtained by capturing care recipient 10.

### (Notification-Time Screen 450)

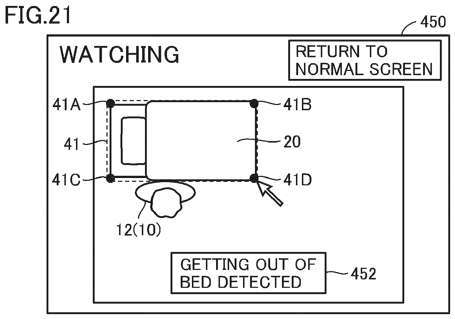

Fig. 21 shows an example of notification-time screen 450. Notification-time screen 450 is a screen displayed when care recipient 10 to be monitored is taking an action which is dangerous while watching system 500 performs the action detection process. Before displaying notification-time screen 450, watching system 500 may inquire of the administrator whether to display notification-time screen 450.

As shown in Fig. 21, based on detecting that care recipient 10 gets out of bed, watching system 500 notifies the caregiver that care recipient 10 gets out of bed. In one aspect, watching system 500 uses a message 352 to notify the caregiver that care recipient 10 gets out of bed. In another aspect, watching system 500 audibly notifies the caregiver that care recipient 10 gets out of bed. In still another aspect, watching system 500 displays an image or video obtained when care recipient 10 getting out of bed is detected. Thus, if watching system 500 should issue notification erroneously, the caregiver can confirm via the image or video an action of care recipient 10 made when the action was detected. This can prevent the caregiver from unnecessarily rushing to care recipient 10.

### [Hardware Configuration of Watching System 500]

An example of a hardware configuration of watching system 500 will be described with reference to Fig. 22. Fig. 22 is a block diagram showing a main hardware configuration of watching system 500.

As shown in Fig. 22, watching system 500 is composed of indoor terminal 100, management server 200, and mobile terminal 300 for a caregiver. Management server 200 is networked to indoor terminal 100 and mobile terminal 300.

In the following, a hardware configuration of indoor terminal 100, management server 200, and mobile terminal 300 will be described.

### (Hardware Configuration of Indoor Terminal 100)

As shown in Fig. 22, indoor terminal 100 includes control device 101, a ROM (Read Only Memory) 102, a RAM (Random Access Memory) 103, a communication interface 104, camera 105, and a storage device 120.

Control device 101 controls indoor terminal 100. Control device 101 is composed for example of at least one integrated circuit. The integrated circuit is composed for example of at least one CPU (Central Processing Unit), at least one ASIC (Application Specific Integrated Circuit), at least one FPGA (Field Programmable Gate Array), or a combination thereof.

An antenna (not shown) or the like is connected to communication interface 104. Indoor terminal 100 communicates data with an external communication device via the antenna. The external communication device includes, for example, management server 200, mobile terminal 300, other communication terminals, and the like. Indoor terminal 100 may be configured to download watching program 122 from the communication terminal.

Camera 105 is, for example, a near infrared camera. The near infrared camera includes an IR (infrared) projector which projects near infrared light. By using the near infrared camera, care recipient 10 can be captured even at night. Alternatively, camera 105 is an ordinary monitoring camera which only receives visible light. Alternatively, as camera 105, a 3D sensor or thermography may be used. Indoor terminal 100 and camera 105 may be integrally configured as shown in Fig. 22 or may be configured separately.

Storage device 120 is, for example, a storage medium such as a hard disk or an external storage device. Storage device 120 stores bed area 41 described above, watching program 122 according to the present embodiment, notification interval information 124 described above, action detection history 126 described above, a notification setting 128 described hereinafter, and the like. Where bed area 41, watching program 122, notification interval information 124, action detection history 126, and notification setting 128 are stored is not limited to storage device 120, and it may be a storage area (for example, cash or the like) of control device 101, ROM 102, RAM 103, an external device (for example, management server 200 or mobile terminal 300), or the like.

It should be noted that watching program 122 may not be provided as a standalone program and instead be incorporated in a portion of any program. In that case, the process according to the present embodiment is implemented in cooperation with the any program. Even such a program that does not include some module does not depart from the gist of watching program 122 according to the present embodiment. Furthermore, a function provided by watching program 122 may partially or entirely be implemented by dedicated hardware. Further, indoor terminal 100 may be configured in such a form as a so-called cloud service in which at least one server executes a part of watching program 122.

### (Hardware Configuration of Management Server 200)

Continuing to refer to Fig. 22, a hardware configuration of management server 200 for a caregiver will be described.

Management server 200 includes a control device 201, a ROM 202, a RAM 203, a communication interface 204, a display interface 205, an operation interface 207, and a storage device 220.

Control device 201 controls management server 200. Control device 201 is composed for example of at least one integrated circuit. The integrated circuit is composed for example of at least one CPU, at least one ASIC, at least one FPGA, or a combination thereof.

An antenna (not shown) or the like is connected to communication interface 204. Management server 200 communicates data with an external communication device via the antenna. The external communication device includes, for example, indoor terminal 100, mobile terminal 300, other communication terminals, and the like. Management server 200 may be configured to download watching program 222 from the communication terminal.

Display interface 205 is connected to display 206 and operates in response to a command issued from control device 101 or the like to send an image signal to display 206 for displaying an image. When display 206 receives from indoor terminal 100 a signal indicating that the care recipient is in danger, display 206 displays a message, an image, or the like accordingly. Preferably, display 206 displays the room number of the care recipient in danger, the name of the care recipient, and what type of danger the care recipient is in (for example, falling). In addition, display 206 displays a setting screen or the like for performing various settings of indoor terminal 100.

Operation interface 207 is, for example, a USB (Universal Serial Bus) terminal, and is connected to an input device 208. Operation interface 207 receives a signal indicating a user operation from input device 208. Input device 208 is, for example, a mouse, a keyboard, a touch panel, or other devices capable of receiving an input operation done by a user.

Storage device 220 is, for example, a storage medium such as a hard disk or an external storage device. Storage device 220 stores watching program 222 according to the present embodiment, history information 224 described above, and the like. Where watching program 222 and history information 224 are stored is not limited to storage device 220, and it may be a storage area (for example, a cache) of control device 201, ROM 202, RAM 203, an external device (for example, indoor terminal 100 and management server 200), or the like.

It should be noted that watching program 222 may not be provided as a standalone program and instead be incorporated in a portion of any program. In that case, the process according to the present embodiment is implemented in cooperation with the any program. Even such a program that does not include some module does not depart from the gist of watching program 222 according to the present embodiment. Furthermore, a function provided by watching program 222 may partially or entirely be implemented by dedicated hardware. Further, indoor terminal 100 may be configured in such a form as a so-called cloud service in which at least two servers execute a part of watching program 222.

### (Hardware Configuration of mobile terminal 300)

Continuing to refer to Fig. 22, a hardware configuration of mobile terminal 300 for a caregiver will be described.

Mobile terminal 300 includes a control device 301, a ROM 302, a RAM 303, a communication interface 304, a display 305, and a storage device 320.

Control device 301 controls mobile terminal 300. Control device 301 is composed for example of at least one integrated circuit. The integrated circuit is composed for example of at least one CPU, at least one ASIC, at least one FPGA, or a combination thereof.

Control device 301 controls mobile terminal 300 by executing various programs such as a watching program 322 according to the present embodiment. In response to a command received to execute watching program 322, control device 301 reads watching program 322 from storage device 320 into ROM 302. RAM 303 functions as a working memory and temporarily stores a variety of types of data necessary for executing watching program 322.

An antenna (not shown) or the like is connected to communication interface 304. Mobile terminal 300 communicates data with an external communication device via the antenna. The external communication device includes, for example, indoor terminal 100, management server 200, and the like. Indoor terminal 100 may be configured to download watching program 322 from the server via the antenna.

When display 305 receives from management server 200 a signal indicating that the care recipient is in danger, display 305 displays a message, an image, or the like accordingly. Preferably, display 305 displays the room number of the care recipient in danger, the name of the care recipient, and what type of danger the care recipient is in (for example, falling). Display 305 overlaps a touchscreen panel (not shown) and receives a variety of types of operations done to mobile terminal 300 through a touching operation.

Storage device 320 is a storage medium such as eMMC (Embedded MultiMediaCard), for example. As an example, storage device 320 stores watching program 322. Where watching program 322 is stored is not limited to storage device 320, and may for example be a cache of control device 301, ROM 302, RAM 303, or another communication device.

### [Sub-Summary]

Thus indoor terminal 100 previously holds notification interval information 124 (see Fig. 4) specifying a notification interval for each combination of actions. When indoor terminal 100 detects a first action targeted for notification and, following the first action, detects a second action targeted for notification, indoor terminal 100 determines from the notification intervals specified in notification interval information 124 a notification interval associated with the combination of the first action and the second action. When a detection interval from the detection of the first action to the detection of the second action is shorter than the determined notification interval, indoor terminal 100 suppresses notification of the second action. Indoor terminal 100 can thus suppress notifying a caregiver of actions associated during a short period of time and thus relieve a burden on the caregiver.

### <Second Embodiment>

### [Outline]

Indoor terminal 100 according to the first embodiment uses single piece of notification interval information 124 to determine whether to suppress an action targeted for notification. Indoor terminal 100 according to a second embodiment uses a plurality of pieces of notification interval information 124 to determine whether to suppress an action targeted for notification.

Indoor terminal 100 according to the second embodiment has a hardware configuration, which is identical to that of indoor terminal 100 according to the first embodiment, and accordingly, will not be described repeatedly.

### [Notification Interval Information 124]

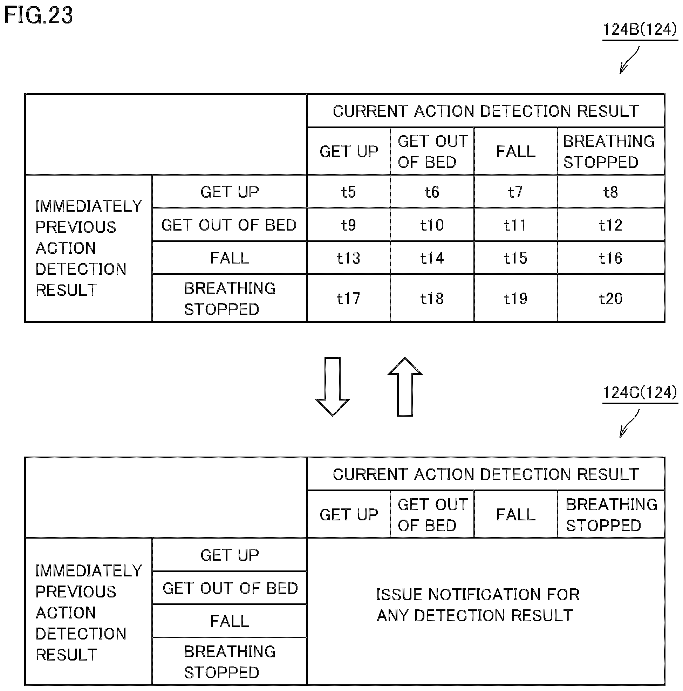

With reference to Fig. 23, notification interval information 124 according to the second embodiment will be described. Fig. 23 shows notification interval information 124B and 124C as an example of notification interval information 124.

Indoor terminal 100 according to the second embodiment holds a plurality of pieces of notification interval information 124B and 124C in advance. Indoor terminal 100 according to the second embodiment switches a setting of the notification interval information from one of notification interval information 124B and 124C to the other depending on the current mode of operation.

More specifically, indoor terminal 100 has a suppression mode and a normal mode as modes of operation. When the current mode of operation is the suppression mode, indoor terminal 100 uses notification interval information 124B. When the current mode of operation is the suppression mode, indoor terminal 100 uses notification interval information 124C.

Notification interval information 124B is as has been described in Fig. 7 and accordingly, will not be described repeatedly. In notification interval information 124C, a notification interval is set to issue notification of any action targeted for notification detected. That is, in notification interval information 124C, notification intervals t5 to t20 are all set to 0 second.

The mode of operation of indoor terminal 100 is switched depending on whether a predetermined condition is satisfied. In an aspect, when a predetermined combination of actions is detected for a short period of time (for example of one second), indoor terminal 100 sets the mode of operation to the suppression mode and sets the setting of the notification interval information to notification interval information 124B. The predetermined combination of actions is, for example, a combination of identical actions (for example, "getting up → getting up"), a combination of actions less likely to occur (for example, "falling → getting out of bed"), and the like. In contrast, when a predetermined action is detected, indoor terminal 100 sets the mode of operation to the normal mode and sets the setting of the notification interval information to notification interval information 124C. The predetermined action is, for example, a care recipient entering and exiting a room.

In another aspect, the mode of operation is switched by a user operation. When the mode of operation is switched to the suppression mode by the user operation, indoor terminal 100 sets the setting of the notification interval information to notification interval information 124B. When the mode of operation is switched to the normal mode by the user operation, indoor terminal 100 sets the setting of the notification interval information to notification interval information 124C.

While in Fig. 23 an example in which notification interval information 124C has its notification intervals all set to "0 second" has been described, notification interval information 124C may have some notification intervals set to "0 second." Notification interval information 124C does not necessarily have its notification intervals all set to "0 second" and notification interval information 124C having a notification interval shorter than those in notification interval information 124B (for example, setting notification interval t6 to minus (-) 10 seconds) suffices.

Furthermore, notification interval information 124 prepared is not limited to the two pieces of notification interval information 124B and 124C, and three or more pieces of notification interval information may be prepared. In that case, indoor terminal 100 has "strong suppression," "moderate suppression," and "weak suppression" as modes of operation, and, depending on the current mode of operation, uses the corresponding notification interval information.

### <Third Embodiment>

### [Outline]

Indoor terminal 100 according to the first embodiment employs a single type of suppression method to suppress notification of an action detected. In contrast, indoor terminal 100 according to a third embodiment employs a plurality of types of suppression methods to suppress notification of an action detected.

Indoor terminal 100 according to the third embodiment has a hardware configuration, which is identical to that of indoor terminal 100 according to the first embodiment, and accordingly, will not be described repeatedly.

### [Notification Setting 128]

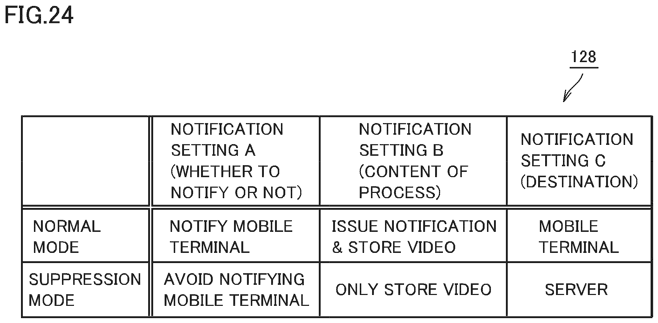

With reference to Fig. 24, a notification setting by indoor terminal 100 according to the third embodiment will be described. Fig. 24 shows a notification setting 128 referred to by indoor terminal 100 according to the third embodiment.

Indoor terminal 100 has a suppression mode and a normal mode as modes of operation. Indoor terminal 100 according to the present embodiment further receives a notification setting for each mode of operation. As an example, indoor terminal 100 receives one of notification settings A to C.

When notification setting A is selected, and the mode of operation is the normal mode and an action targeted for notification is detected, indoor terminal 100 notifies a caregiver's mobile terminal 300 of that action. When notification setting A is selected, and the mode of operation is the suppression mode and an action targeted for notification is detected, indoor terminal 100 does not notify the caregiver's mobile terminal 300 of that action. Thus, when notification setting A is selected, indoor terminal 100 switches whether to issue notification or not depending on the mode of operation.

When notification setting B is selected, and the mode of operation is the normal mode and an action targeted for notification is detected, indoor terminal 100 notifies the caregiver's mobile terminal 300 of that action and also stores a video showing that action. When notification setting B is selected, and the mode of operation is the suppression mode and an action targeted for notification is detected, indoor terminal 100 does not notify the caregiver's mobile terminal 300 of that action and stores a video showing that action. Thus, when notification setting B is selected, indoor terminal 100 changes content of notification in quality depending on the mode of operation.

When notification setting C is selected, and the mode of operation is the normal mode and an action targeted for notification is detected, indoor terminal 100 notifies the caregiver's mobile terminal 300 of that action. When notification setting C is selected, and the mode of operation is the suppression mode and an action targeted for notification is detected, indoor terminal 100 notifies management server 200 of that action. Thus, when notification setting C is selected, indoor terminal 100 changes a destination for notification depending on the mode of operation.

### <Summary>

According to one aspect, a watching system capable of issuing notification of an action of a person to be watched comprises: an action detection unit capable of detecting a plurality of types of actions of the person; a storage unit for storing information specifying a notification interval for each combination of actions; a determination unit for determining, when the action detection unit detects that the person takes a first action targeted for notification and, following the first action, detects that the person takes a second action targeted for notification, a notification interval from notification intervals specified in the information, that is associated with a combination of the first action and the second action; and a notification control unit for suppressing notification of the second action when a detection interval from the detection of the first action to the detection of the second action is shorter than the notification interval determined by the determination unit.

Preferably, the notification control unit issues notification indicating that the second action is detected when the second action is detected and the detection interval is longer than the notification interval determined by the determination unit, and the notification control unit does not issue notification indicating that the second action is detected when the second action is detected and the detection interval is shorter than the notification interval determined by the determination unit.

Preferably, the notification control unit issues notification indicating that the first action is detected when the first action is detected.

Preferably, when the detection interval is longer than the notification interval determined by the determination unit, the notification control unit transmits information representing the first action and information representing the second action to a communication terminal together with the notification.

Preferably, the notification control unit changes a manner of notification of the second action depending on whether the detection interval is longer than the notification interval determined by the determination unit.

It should be understood that the embodiments disclosed herein have been described for the purpose of illustration only and in a non-restrictive manner in any respect. The scope of the present invention is defined by the terms of the claims, rather than the description above, and is intended to include any modifications within the meaning and scope equivalent to the terms of the claims.

### Reference Signs List

10: caregiver; 11: care recipient; 12: person area; 20: bed; 30, 30A-30E, 35B: input image; 35A: background image; 36: background subtracted image; 41: bed area; 41A to 41D point; 100: indoor terminal; 101, 201, 301: control device; 102, 202, 302: ROM; 103,203, 303: RAM; 104, 204, 304: communication interface; 105: camera; 120, 220, 320: storage device; 122, 222, 332: watching program; 124, 124A-124C: notification interval information; 126: action detection history; 128: notification setting; 150: person detection unit; 152: action detection unit; 154: determination unit; 156: clock unit; 158: notification control unit; 200: management server; 205: display interface; 206, 305: display; 207: operation interface; 208: input device; 224: history information; 250: communication unit; 252: reception unit; 300: mobile terminal; 351: notification unit; 352: message; 410: main screen; 412, 414, 422, 424: button; 420: setting mode top screen; 430: area setting screen; 432: pointer; 440: normal-time screen; 450: notification-time screen; 500: watching system.

## Claims

1. A watching system capable of issuing notification of an action of a person to be watched, comprising:
an action detection unit capable of detecting a plurality of types of actions of the person;
a storage unit for storing information specifying a notification interval for each combination of actions;
a determination unit for determining, when the action detection unit detects that the person takes a first action targeted for notification and, following the first action, detects that the person takes a second action targeted for notification, a notification interval from notification intervals specified in the information, that is associated with a combination of the first action and the second action; and
a notification control unit for suppressing notification of the second action when a detection interval from the detection of the first action to the detection of the second action is shorter than the notification interval determined by the determination unit.

2. The watching system according to claim 1, wherein
the notification control unit issues notification indicating that the second action is detected when the second action is detected and the detection interval is longer than the notification interval determined by the determination unit, and
the notification control unit does not issue notification indicating that the second action is detected when the second action is detected and the detection interval is shorter than the notification interval determined by the determination unit.

3. The watching system according to claim 1 or 2, wherein the notification control unit issues notification indicating that the first action is detected when the first action is detected.

4. The watching system according to claim 3, wherein when the detection interval is longer than the notification interval determined by the determination unit, the notification control unit transmits information representing the first action and information representing the second action to a communication terminal together with the notification.

5. The watching system according to claim 1, wherein the notification control unit changes a manner of notification of the second action depending on whether the detection interval is longer than the notification interval determined by the determination unit.

6. A watching device capable of issuing notification of an action of a person to be watched, comprising:
an action detection unit capable of detecting a plurality of types of actions of the person;
a storage unit for storing information specifying a notification interval for each combination of actions;
a determination unit for determining, when the action detection unit detects that the person takes a first action targeted for notification and, following the first action, detects that the person takes a second action targeted for notification, a notification interval from notification intervals specified in the information, that is associated with a combination of the first action and the second action; and
a notification control unit for suppressing notification of the second action when a detection interval from the detection of the first action to the detection of the second action is shorter than the notification interval determined by the determination unit.

7. A watching method capable of detecting a plurality of types of actions of a person to be watched, for issuing notification of a detected action, comprising:
preparing information specifying a notification interval for each combination of actions;
determining, when the person taking a first action targeted for notification is detected and, following the first action, the person taking a second action targeted for notification is detected, a notification interval from notification intervals specified in the information, that is associated with a combination of the first action and the second action; and
suppressing notification of the second action when a detection interval from the detection of the first action to the detection of the second action is shorter than the notification interval determined in the step of determining.

8. A watching program capable of detecting a plurality of types of actions of a person to be watched, and executed by a computer for issuing notification of a detected action, the watching program causing the computer to perform the steps of:
preparing information specifying a notification interval for each combination of actions;
determining, when the person taking a first action targeted for notification is detected and, following the first action, the person taking a second action targeted for notification is detected, a notification interval from notification intervals specified in the information, that is associated with a combination of the first action and the second action; and
suppressing notification of the second action when a detection interval from the detection of the first action to the detection of the second action is shorter than the notification interval determined in the step of determining.
